# EUROPÄISCHE PATENTSCHRIFT

(11) **EP 0 476 403 B1**
(45) Veröffentlichungstag und Bekanntmachung des Hinweises auf die Patenterteilung: **28.02.1996**
(21) Anmeldenummer: 91114705.6
(22) Anmeldetag: 31.08.1991
(51) Int. Cl.: C07D 251/42, A01N 47/36

(54) **Herbizide[[(1,3,5-Triazin-2-yl)aminocarbonyl]aminosulfonyl]benzoesäureester, Verfahren zu ihrer Herstellung und ihre Verwendung**
Herbicidal(((1,3,5-triazine-2-yl)aminocarbonyl)aminosulfonyl)benzoeacidesters, method for their preparation and their use
Esters d'acides (((triazine-1,3,5 yl-2)aminocarbonyl)aminosulfonyl)benzoiques herbicides, procédé pour leur préparation et leur utilisation

(30) Priorität: 18.09.1990 DE 4029484
(43) Veröffentlichungstag der Anmeldung: 25.03.1992
(73) Patentinhaber: BASF Aktiengesellschaft, D-67063 Ludwigshafen (DE)
(72) Erfinder: Mayer, Horst, Dr., W-6700 Ludwigshafen (DE); Hamprecht, Gerhard, Dr., W-6940 Weinheim (DE); Westphalen, Karl-Otto, Dr., W-6720 Speyer (DE); Walter, Helmut, Dr., W-6719 Obrigheim (DE); Gerber, Matthias, Dr., W-6704 Mutterstadt (DE)

(56) Entgegenhaltungen:
- EP-A- 0 007 687
- EP-A- 0 030 138
- EP-A- 0 101 670
- EP-A- 0 162 723
- EP-A- 0 382 437
- EP-A- 0 388 873
- WO-A-89/02595
- WO-A-89/09214
- CHEMICAL ABSTRACTS, Band 107, Nr. 19, 9. November 1987, Columbus, Ohio, USA DU PONT DE NEMOURS; E.L. AND CO. "Preparation of 2-(((2- -pyrimidinyl and 1,3,5- tria- zin-2-yl)ureido)sulfonyl) benzoates as herbicides and plant growth regulators." Seite 729, Spalte 1, Zusammenfassung-Nr. 176 061n

## Beschreibung

Die vorliegende Erfindung betrifft [[(1,3,5-Triazin-2-yl)aminocarbonyl]aminosulfonyl]benzoesäureester der allgemeinen Formel I 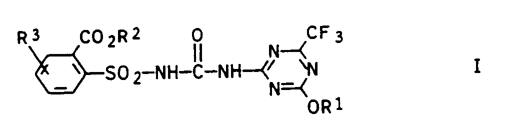
in der die Substituenten folgende Bedeutung haben:
- R¹: eine Methyl- oder Ethylgruppe;
- R²: i-Propyl;
- R³: Wasserstoff, Fluor, Chlor, Methyl oder Methoxy
sowie deren landwirtschaftlich brauchbare Salze.

Des weiteren betrifft die Erfindung ein Verfahren zur Herstellung der Verbindungen I sowie ihre Verwendung als herbizide Mittel.

Die EP-A 7687, die EP-A 30 138 und die EP-A 57 546 betreffen Sulfonylharnstoffe mit herbizider Wirkung, deren allgemeine Formel die eingangs definierten Verbindungen I umfaßt.

Während in EP-A 57 546 als nächstliegende Strukturen nur 1,3,5-Triazin-N-oxid-Derivate I' 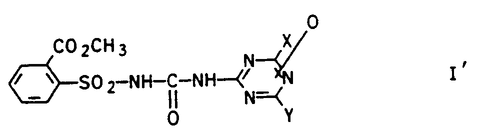
beschrieben sind, in denen die Reste u.a. folgende Bedeutung haben:
- X und Y: unabhängig voneinander Methyl oder Methoxy und
- O: Sauerstoff in 1-, 3- oder 5-Position des Triazinylrings,
liegt bei der EP-A 30 138 der Schwerpunkt auf der Variation des Carbonsäureesters in 2-Position zur Sulfonamid-Gruppe. Halogenalkylsubstituenten für den Triazin-Teil sind an dieser Stelle nicht vorbeschrieben.

In der EP-A 162 723 werden Sulfonylharnstoffe beschrieben, die einen Triazinring aufweisen können, der unter anderem Fluoralkoxy-Substituenten tragen kann.

Lediglich in der EP-A 7687 wird ein Triazinylderivat mit Halogenalkylrest beschrieben (I''). 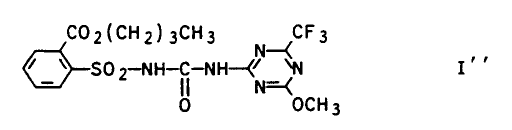
In der EP-A 388 873 werden [[(1,3,5-Triazin-2-yl)aminocarbonyl]-aminosulfonyl]benzoesäureester der Formel I beschrieben, in der R² für eine Methyl- oder Ethylgruppe steht und R³ Wasserstoff, Fluor oder Chlor bedeutet.

Der Erfindung lag die Aufgabe zugrunde, Sulfonylharnstoffe zu synthetisieren, die gegenüber den bekannten Vertretern dieser Herbizid-Klasse verbesserte Eigenschaften aufweisen.

Entsprechend dieser Aufgabe wurden die eingangs definierten [[(1,3,5-Triazin-2-yl)aminocarbonyl]aminosulfonyl]benzoesäureester der Formel I gefunden.

Die erfindungsgemäßen Sulfonylharnstoffe der Formel I sind auf verschiedenen Wegen zugänglich, die in der Literatur beschrieben sind. Beispielhaft seien besonders vorteilhafte Wege (A-C) im folgenden näher erläutert. 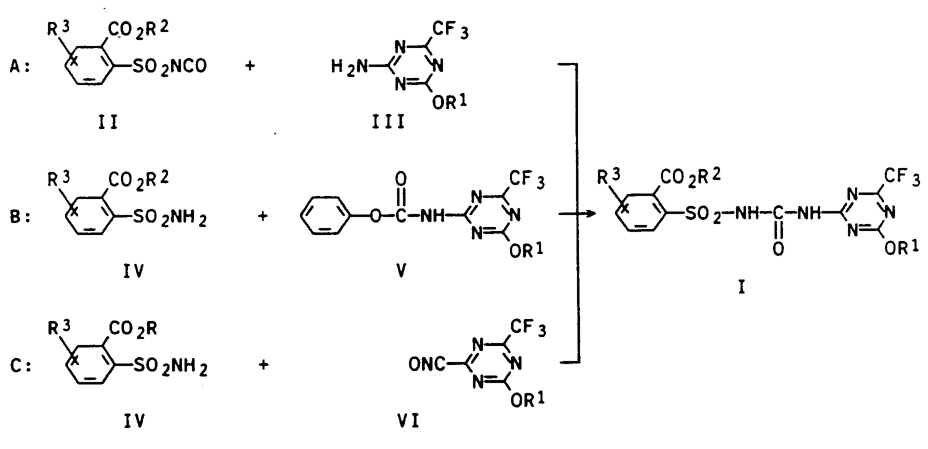
A: Man setzt ein Sulfonylisocyanat II in an sich bekannter Weise (EP-A-162 723) mit ungefähr der stöchiometrischen Menge eines 2-Amino-1,3,5-triazin-derivats III bei einer Temperatur von 0 bis 120°C, vorzugsweise 10 bis 100°C um. Die Reaktion kann unter Normaldruck oder unter Druck (bis 50 bar), vorzugsweise bei 1 bis 5 bar, kontinuierlich oder diskontinuierlich durchgeführt werden.
   Zweckmäßigerweise verwendet man für die Umsetzungen unter den jeweiligen Reaktionsbedingungen inerte Lösungs- und Verdünnungsmittel. Als Lösungsmittel kommen beispielsweise Halogenkohlenwasserstoffe, insbesondere Chlorkohlenwasserstoffe, z.B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol, 1,2,4-Trichlorbenzol; Ether, z.B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, β,β'-Dichlordiethylether; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester, z.B. Ethylacetat, Acetessigester, Isobutylacetat; Amide, z.B. Formamid, Methylformamid, Dimethylformamid; Ketone, z.B. Aceton, Methylethylketon und entsprechende Gemische in Betracht. Zweckmäßig verwendet man das Lösungsmittel in einer Menge von 100 bis 2000 Gew.%, vorzugsweise von 200 bis 700 Gew.%, bezogen auf den Ausgangsstoff II.
   Die zur Umsetzung benötigte Verbindung II wird im allgemeinen in etwa äquimolaren Mengen (mit einem Über- oder Unterschuß von z.B. 0 bis 20 %), bezogen auf den jeweiligen Ausgangsstoff III eingesetzt. Man kann den Ausgangsstoff III in einem der vorgenannten Verdünnungsmittel vorlegen und dann den Ausgangsstoff II zugeben.
   Zweckmäßigerweise wird das Verfahren zur Herstellung der neuen Verbindungen jedoch so durchgeführt, daß man den Ausgangsstoff II, gegebenenfalls in einem der vorgenannten Verdünnungsmittel, vorlegt und dann den Ausgangsstoff III zugibt.
   Zur Beendigung der Umsetzung rührt man nach der Zugabe der Komponenten noch 20 Minuten bis 24 Stunden bei 0 bis 120°C, vorzugsweise 10 bis 100°C, nach.
   Als Reaktionsbeschleuniger kann man vorteilhafterweise ein tertiäres Amin, z.B. Pyridin, α,β,γ-Picolin, 2,4-, 2,6-Lutidin, 2,4,6-Collidin, p-Dimethylaminopyridin, Trimethylamin, Triethylamin, Tri(n-propyl)amin, 1,4-Diazabicyclo[2.2.2]octan [DABCO] oder 1,8-Diazabicylco-[5.4.0]-undec-7-en in einer Menge von 0.01 bis 1 mol pro Mol Ausgangsstoff II verwenden.
   Aus dem Reaktionsgemisch wird der Endstoff I in üblicher Weise, z.B. nach Abdestillieren von Lösungsmittel oder direkt durch Absaugen isoliert. Der verbleibende Rückstand kann noch mit Wasser bzw. verdünnter Säure zur Entfernung basischer Verunreinigungen gewaschen werden. Man kann jedoch auch den Rückstand in einem mit Wasser nicht mischbaren Lösungsmittel lösen und wie beschrieben waschen. Die gewünschten Endstoffe fallen hierbei in reiner Form an, gegebenenfalls können sie durch Umkristallisation, Rühren in einem organischen Lösungsmittel, das die Verunreinigungen aufnimmt oder Chromatographie gereinigt werden.
   Bevorzugt führt man diese Umsetzung in Acetonitril, Methyl-tert.-butylether, Toluol oder Methylenchlorid in Anwesenheit von 0 bis 100 Moläquivalenten, vorzugsweise 0 bis 50 Moläquivalenten eines tertiären Amins wie 1,4-Diazabicyclo-[2,2,2]-octan oder Triethylamin durch.
B: Man setzt ein Sulfonamid der Formel IV in an sich bekannter Weise (EP-A-141 777 und EP-A-101 670) in einem inerten organischen Lösungsmittel mit ungefähr der stöchiometrischen Menge eines Phenylcarbamats V bei einer Temperatur von 0 bis 120°C, vorzugsweise 20 bis 100°C um. Die Reaktion kann bei Normaldruck oder unter Druck (bis 50 bar), vorzugsweise bei 1 bis 5 bar, kontinuierlich oder diskontinuierlich durchgeführt werden.
   Es können hierbei Basen wie tertiäre Amine zugesetzt werden, die die Reaktion beschleunigen und die Produktqualität verbessern. Geeignete Basen sind hierfür die unter A angegebenen, insbesondere Triethylamin, 2,4,6-Collidin, 1,4-Diazabicyclo[2.2.2]octan [DABCO] oder 1,8-Diazabicyclo[5.4.0]undec-7-en (DBU), in einer Menge von 0,01 bis 1 mol pro Mol Ausgangsstoff IV.
   Zweckmäßigerweise verwendet man als Löse- oder Verdünnungsmittel die unter A angegebenen.
   Man verwendet das Lösungsmittel in einer Menge von 100 bis 2000 Gew.%, vorzugsweise von 200 bis 700 Gew.%, bezogen auf das Edukt IV.
   Die zur Umsetzung benötigte Verbindung IV wird im allgemeinen in etwa äquimolaren Mengen (mit einem Über- oder Unterschuß von z.B. 0 bis 20 %), bezogen auf die jeweiligen Ausgangsstoffe V eingesetzt. Man kann den Ausgangsstoff V in einem der vorgenannten Verdünnungsmittel vorlegen und dann den Ausgangsstoff IV zugeben.
   Man kann jedoch auch den Ausgangsstoff IV In einem der genannten Lösungsmittel vorlegen und dann das Carbamat V zugeben. In beiden Fällen kann als Katalysator vor oder während der Reaktion eine der genannten Basen zugesetzt werden.
   Zur Beendigung der Umsetzung rührt man nach der Zugabe der Komponenten noch 20 Minuten bis 24 Stunden bei 0 bis 120°C, vorzugsweise 10 bis 100°C, insbesondere 20 bis 80°C, nach.
   Man isoliert die Sulfonylharnstoffe der Formel I aus dem Reaktionsgemisch mit den üblichen Methoden, wie unter A beschrieben.
C: Man setzt ein Sulfonamid der Formel IV in an sich bekannter Weise (EP-A-234 352) in einem inerten organischen Lösungsmittel mit ungefähr der stöchiometrischen Menge eines Isocyanates VI zu einer Temperatur von 0 bis 150°C, vorzugsweise 10 bis 100°C um. Die Reaktion kann bei Normaldruck oder unter Druck (bis 50 bar), vorzugsweise bei 1 bis 5 bar, kontinuierlich oder diskontinuierlich durchgeführt werden.
   Es können hierbei vor oder während der Reaktion Basen wie tertiäre Amine zugesetzt werden, die die Reaktionsbeschleuniger und die Produktqualität verbessern. Geeignete Basen sind hierfür die unter A angegebenen, insbesondere Triethylamin oder 2,4,6-Collidin, in einer Menge von 0,01 bis 1 mol pro Mol Ausgangsstoff IV.
   Zweckmäßigerweise verwendet man als Lösungsmittel die unter A angegebenen. Man setzt das Lösungsmittel in einer Menge von 100 bis 2000 Gew.%, vorzugsweise von 200 bis 700 Gew.%, bezogen auf das Edukt IV ein.
   Die zur Umsetzung benötigte Verbindung IV wird im allgemeinen in etwa äquimolaren Mengen (mit einem Über- oder Unterschuß von z.B. 0 bis 20 %), bezogen auf die Edukte VI eingesetzt. Man kann den Ausgangsstoff VI in einem der genannten Verdünnungsmittel vorlegen und dann den Ausgangsstoff IV zugeben.
   Man kann aber auch das Sulfonamid vorlegen und dann das Isocyanat VI zugeben.
   Zur Beendigung der Umsetzung rührt man nach der Zugabe der Komponenten noch 20 Minuten bis 24 Stunden bei 0 bis 120°C, vorzugsweise 10 bis 100°C, insbesondere 20 bis 80°C, nach.
   Das Endprodukt I kann aus dem Reaktionsgemisch in der üblichen Weise, wie unter A: beschrieben, gewonnen werden.

Die als Edukte verwendeten Sulfonylisocyanate der Formel II wurden nach oder in Analogie zu Literaturmethoden (z.B. EP-A-7687) gewonnen.

Carbamate der Formel V sind nach oder in Analogie zu bekannten Umsetzungen (z.B. EP-A-141 777) zugänglich, sie lassen sich aber auch aus den entsprechenden Isocyanaten der Formel VI durch Umsetzung mit Phenol, herstellen.

Die Salze der Verbindungen I sind in an sich bekannter Weise zugänglich (EP-A-304 282, US-A 4,599,412). Man erhält sie durch Deprotonierung der entsprechenden Sulfonylharnstoffe I in Wasser oder einem inerten organischen Lösungsmittel bei Temperaturen von -80°C bis 120°C, vorzugsweise 0°C bis 60°C in Gegenwart einer Base.

Geeignete Basen sind beispielsweise Alkali- oder Erdalkalimetallhydroxide, -hydride, -oxide oder -alkoholate wie Natrium-, Kalium- und Lithiumhydroxid, Natriummethanolat, -ethanolat und -tert.-butanolat, Natrium- und Calziumhydrid und Calziumoxid.

Als Lösungsmittel kommen beispielsweise neben Wasser auch Alkohole wie Methanol, Ethanol und tert.-Butanol, Ether wie Tetrahydrofuran und Dioxan, Acetonitril, Dimethylformamid, Ketone wie Aceton und Methylethylketon und auch halogenierte Kohlenwasserstoffe in Betracht.

Die Deprotonierung kann bei Normaldruck oder bei Drucken bis 50 bar, vorzugsweise bei Normaldruck bis 5 bar Überdruck durchgeführt werden.

Die als Ausgangsstoffe der Formel IV benötigten Sulfonamide lassen sich aus den entsprechenden Anthranilsäureestern durch Meerwein-Reaktion und anschließende Umsetzung mit Ammoniak herstellen (Houben-Weyl, 9, 557f (1955)).

Die Edukte 2-Amino-4-methoxy-6-trifluormethyl-1,3,5-triazin und 2-Amino-4-ethoxy-6-trifluormethyl-1,3,5-triazin sind literaturbekannt (Yakugaku Zasshi 95, 499 (1975)).

Die Verbindungen I bzw. die sie enthaltenden herbiziden Mittel sowie deren umweltverträgliche Salze von Alkalimetallen und Erdalkalimetallen können in Kulturen wie Weizen Schadpflanzen sehr gut bekämpfen, ohne die Kulturpflanzen zu schädigen, ein Effekt, der vor allem auch bei niedrigen Aufwandmengen auftritt. Sie können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen u.a. Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,1 und 95 Gew.%, vorzugsweise zwischen 0,5 und 90 Gew.%, Wirkstoff.

Beispiele für Formulierungen sind:
I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.
II. 20 Gewichtsteile der Verbindung Nr. 1 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
III. 20 Gewichtsteile der Verbindung Nr. 1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. 1 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
V. 20 Gewichtsteile des Wirkstoffs Nr. 1 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.
VI. 3 Gewichtsteile des Wirkstoffs Nr. 1 werden mit 97 Gewichtsteilen feinteiligem Kaolin vermischt. Man erhält auf diese Weise ein Stäubemittel, das 3 Gew.% des Wirkstoffs enthält.
VII. 30 Gewichtsteile des Wirkstoffs Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 20 Gewichtsteile des Wirkstoffs Nr. 1 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0 kg/ha, vorzugsweise 0,01 bis 2,0 kg/ha.

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

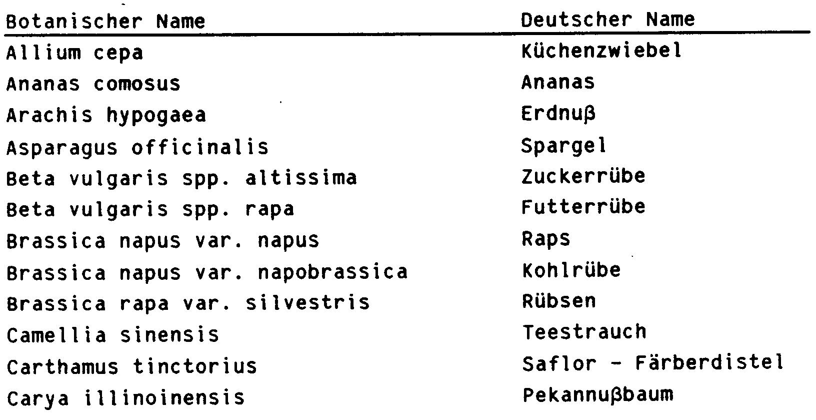

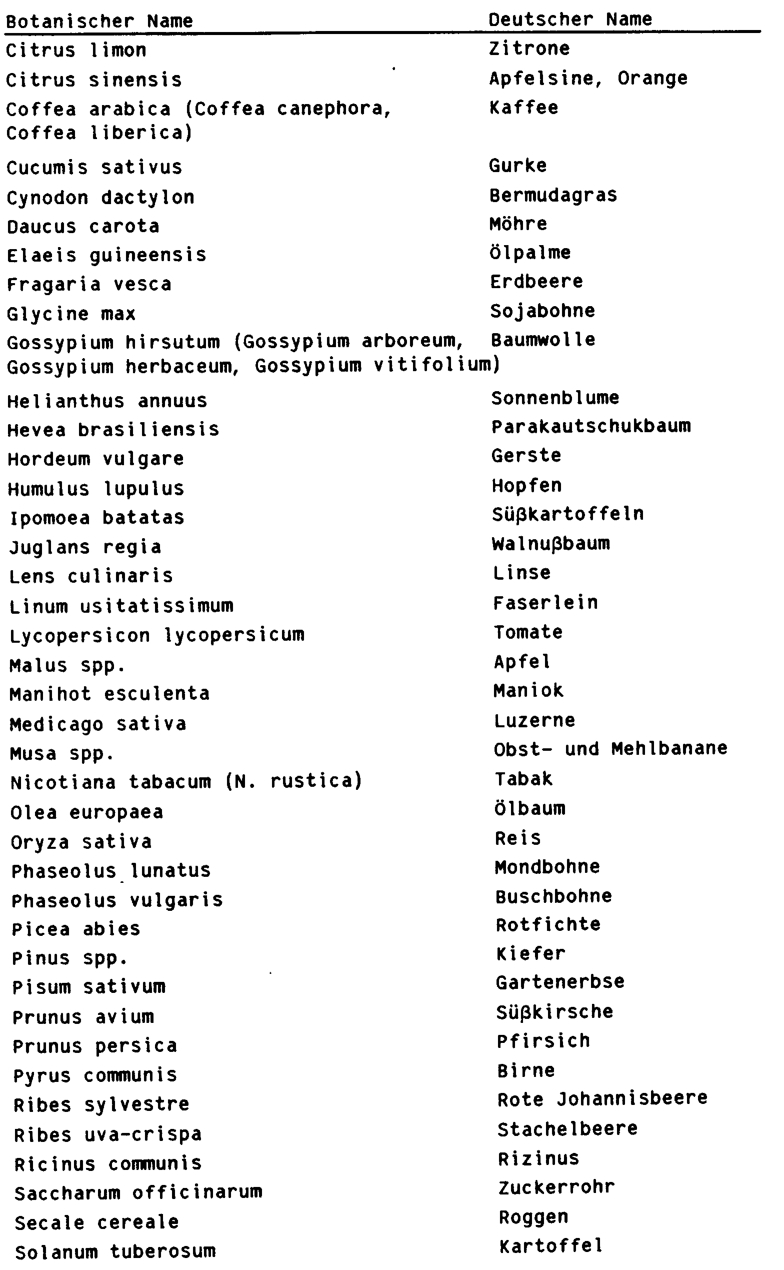

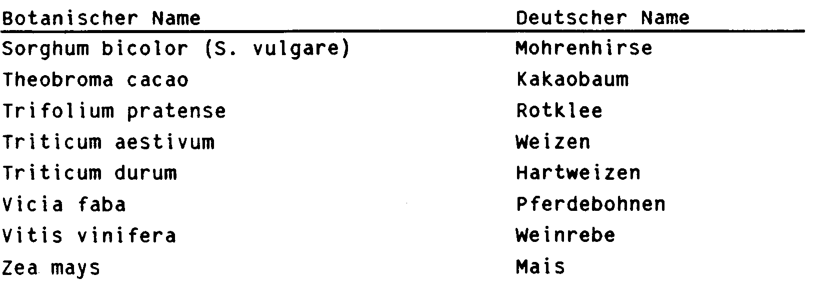

Zur Verbreiterung des Wirkungsspektrums und zur Erzielung synergistischer Effekte können die Triazinyl-substituierten Sulfonylharnstoffe der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiocarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Sulfonylharnstoffe, Phenyloxy- bzw. Heteroaryloxy-phenoxypropionsäuren sowie deren Salze, Ester und Amide und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Nachstehend sind Beispiele für die Synthese der Verbindungen I wiedergegeben.

### Beispiel 1

### [[(4-Methoxy-6-trifluormethyl-1,3,5-triazin-2-yl)aminocarbonyl]aminosulfonyl]-benzoesäureisopropylester

Eine Lösung von 3,9 g 2-Amino-4-methoxy-6-trifluormethyl-1,3,5-triazin (20 mmol) in 150 ml 1,2-Dichlorethan wird tropfenweise innerhalb von 5 min mit einer Lösung von 5,4 g 2-(i-Propoxycarbonyl)benzolsulfonylisocyanat (20 mmol) in 5,4 g 1,2-Dichlorethan bei 25°C versetzt. Man rührt 16 h bei 25°C, dann 2 h bei 50°C. Nach Entfernen der flüchtigen Bestandteile im Wasserstrahlvakuum bei 60°C wird der Rückstand mit einem Ethanol-/Pentan-Gemisch gerührt, worauf Kristallisation eintritt. Das Produkt wird abgesaugt und im Wasserstrahlvakuum bei 50°C getrocknet. Man isoliert 6,1 g der Titelverbindung (13 mmol; 66 % d.Th.) mit Fp. 182-185°C.

### Beispiel 2

### Calcium[bis[[(4-methoxy-6-trifluormethyl-1,3,5-triazin-2-yl)aminocarbonyl]aminosulfonyl]benzoesäure(i-propyl)ester]

Man trägt 67 mg CaH₂ (1,6 mmol) in 50 ml Methanol ein und rührt 1 Stunde bei 50°C. Nach Zugabe von 1,5 g [[(4-Methoxy-6-trifluormethyl-1,3,5-triazin-2-yl)aminocarbonyl]aminosulfonyl]benzoesäure(i-propyl)ester (3,2 mmol) rührt man 1 h bei 25°C, wobei eine homogene Lösung erhalten wird. Nach Entfernen der flüchtigen Bestandteile im Wasserstrahlvakuum bei 80°C erhält man die Titelverbindung in quantitativer Ausbeute mit einem Zersetzungspunkt von 160°C.

### Beispiel 3

### 4-Chlor-2[[(4-methoxy-6-trifluormethyl-1,3,5-triazin-2-yl)aminocarbonyl]aminosulfonyl]benzoesäure(i-propyl)ester

Eine Lösung von 3,9 g 2-Amino-4-methoxy-6-trifluormethyl-1,3,5-triazin (20 mmol) in 150 ml 1,2-Dichlorethan wird mit 19,6 g einer Lösung von 6,1 g 5-Chlor-2(i-propoxycarbonyl)benzolsulfonyl-isocyanat (20 mmol) in 1,2-Dichlorethan bei 25°C tropfenweise versetzt. Man rührt 16 h bei 25°C, dann 3 h bei 50°C. Nach Entfernen der flüchtigen Bestandteile im Wasserstrahlvakuum bei 60°C wird der Rückstand mit 200 ml eines tert.-Butylmethylether-/Ether-Gemisches extrahiert. Die Lösungsmittel werden im Wasserstrahlvakuum bei 50°C abdestilliert und der Rückstand in wenig i-Propanol gerührt. Das feste Produkt wird abgesaugt und im Wasserstrahlvakuum bei 40°C getrocknet. Man erhält so 7,2 g der Titelverbindung (14 mmol; 70 % d.Th.) mit Fp. 160-163°C.

### Beispiel 4

### Natrium[4-chlor-2-[[(4-methoxy-6-trifluormethyl-1,3,5-triazin-2-yl)aminocarbonyl]aminosulfonyl]benzoesäure(i-propyl)ester]

Man tropft 0,7 g einer Natriummethanolatlösung (30 gew.%ig in Methanol, 3,9 mmol) bei 25°C zu einer Lösung von 1,9 g 4-Chlor-2-[[(4-methoxy-6-trifluormethyl-1,3,5-triazin-2-yl)aminocarbonyl]aminosulfonyl]benzoesäure(i-propyl)ester (3,8 mmol) in 30 ml Methanol, rührt 10 min bei 25°C und entfernt die flüchtigen Bestandteile bei 60°C im Wasserstrahlvakuum. Man erhält so die Titelverbindung in quantitativer Ausbeute mit einem Zersetzungspunkt von 175°C.

Die in der nachfolgenden Tabelle 1 genannten Wirkstoffe werden auf analogem Herstellungsweg erhalten.

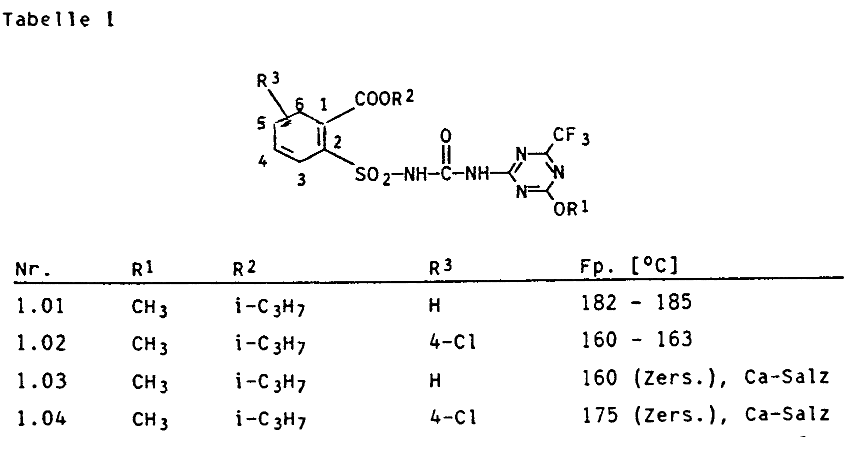

In entsprechender Weise können die in Tabelle 2 aufgeführten Verbindungen hergestellt werden.

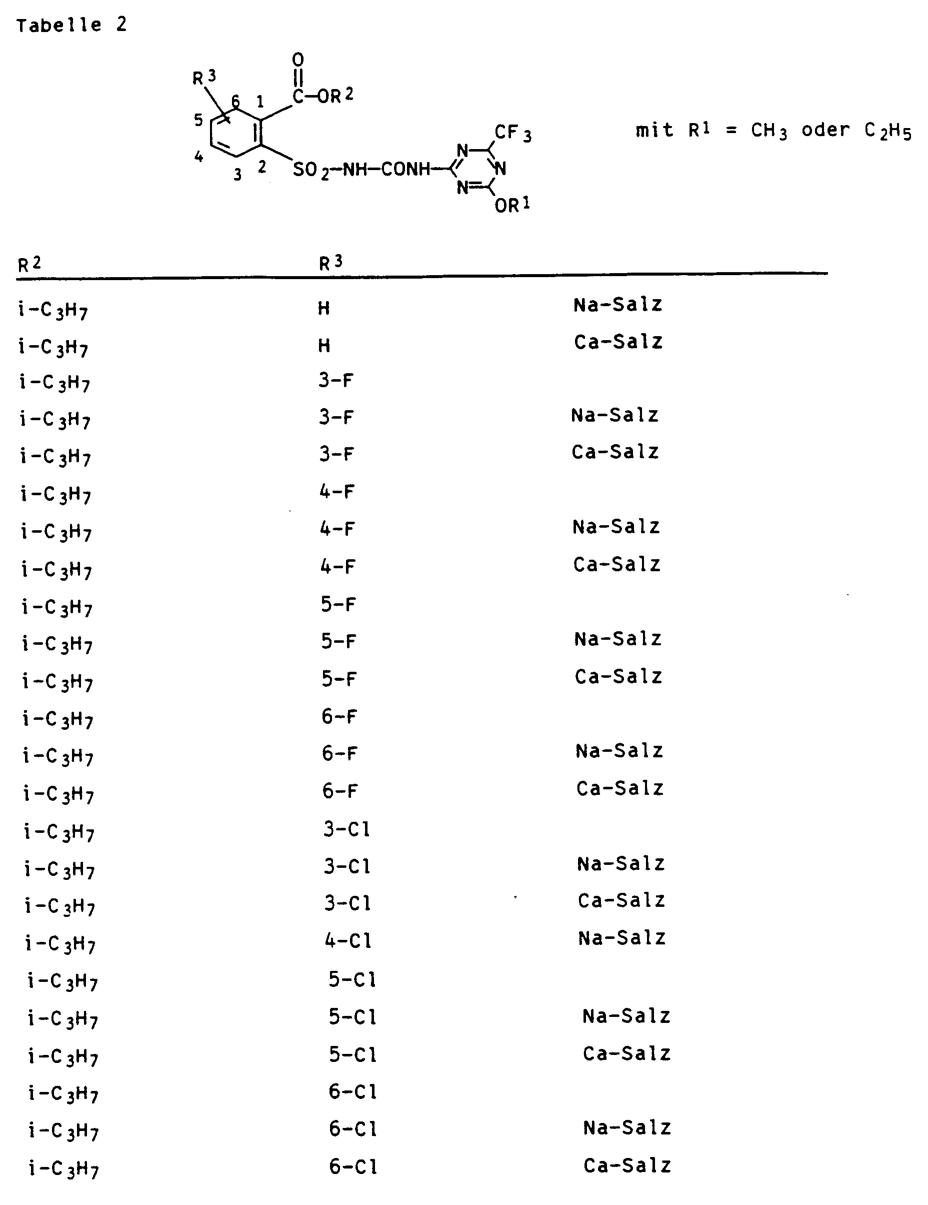

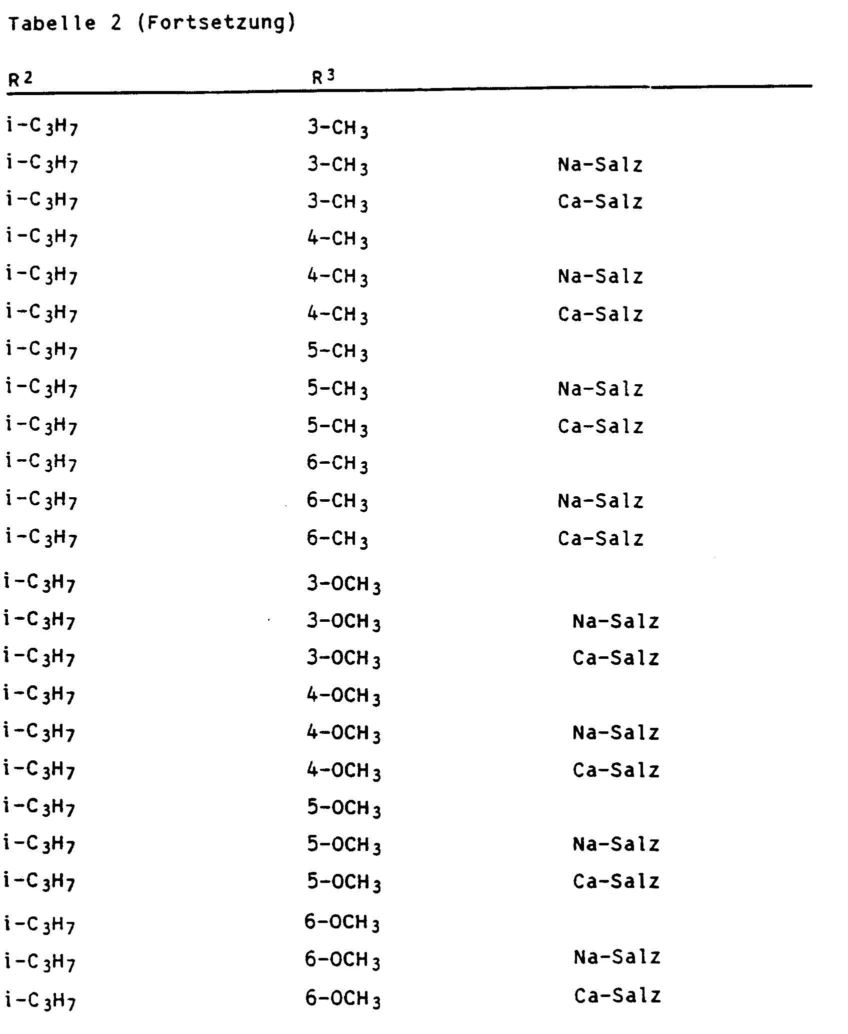

### Anwendungsbeispiele:

Die herbizide Wirkung der [[(1,3,5-Triazin-2-yl)aminocarbonyl]aminosulfonyl]benzoesäureester der Formel I auf das Wachstum der Testpflanzen wird durch folgende Gewächshausversuche gezeigt.

Als Kulturgefäße dienen Plastikblumentöpfe mit 300 cm³ Inhalt und lehmigem Sand mit etwa 3,0 % Humus als Substrat. Die Samen der Testpflanzen werden nach Arten getrennt flach eingesät.

Zum Zwecke der Nachauflaufbehandlung werden entweder direkt gesäte oder in den gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung beträgt 0,06 kg/ha a.S. (aktive Substanz).

Die Versuchsgefäße werden im Gewächshaus aufgestellt, wobei für wärmeliebende Arten wärmere Bereiche (20 bis 35°C) und für solche gemäßigter Klimate 10 bis 20°C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Lateinischer Name | Deutscher Name | Abkürzung |
|---|---|---|
| Amaranthus retroflexus | Zurückgekrümmter Fuchsschwanz | AMARE |
| Cyperus iria | - | CYPIR |
| Galium aparine | Klettenlabkraut | GALAP |
| Ipomoea spp. | Prunkwindearten | IPOSS |
| Polygonum persicaria | Flohknöterich | POLPE |
| Triticum aestivum | Sommerweizen | TRZAS |
| Veronica spp. | Ehrenpreisarten | VERSS |

Mit 0,06 kg/ha a.S. im Nachauflaufverfahren eingesetzt lassen sich mit dem Beispiel 1 breitblättrige unerwünschte Pflanzen sehr gut bekämpfen, bei gleichzeitiger hervorragender Selektivität in der Kulturpflanze Weizen.

In der folgenden Tabelle I sind Ergebnisse aus biologischen Untersuchungen zusammengestellt, in denen der erfindungsgemäße Wirkstoff Nr. 1 mit der aus EP-A 7687 bekannten Vergleichsverbindung A verglichen wurden.

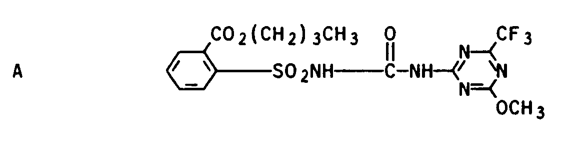

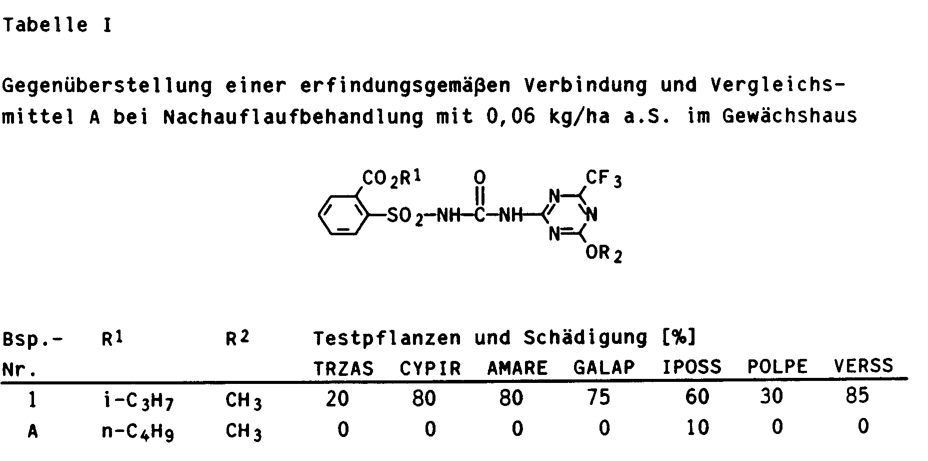

## Patentansprüche

1. [[(1,3,5-Triazin-2-yl)aminocarbonyl]aminosulfonyl]benzoesäureester der allgemeinen Formel I 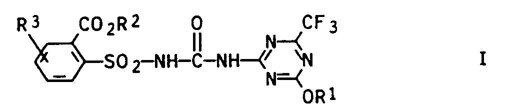 in der die Substituenten folgende Bedeutung haben:
R¹ eine Methyl- oder Ethylgruppe;
R² i-Propyl;
R³ Wasserstoff, Fluor, Chlor, Methyl oder Methoxy
sowie deren landwirtschaftlich brauchbare Salze.

2. Verfahren zur Herstellung der [[(1,3,5-Triazin-2-yl)aminocarbonyl]aminosulfonyl]benzoesäureester der Formel I gemäß Anspruch 1, dadurch gekennzeichnet, daß man ein Sulfonylisocyanat II 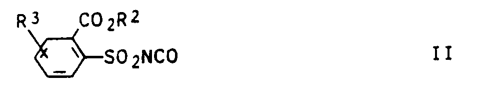 in an sich bekannter Weise in einem inerten organischen Lösungsmittel mit der ungefähr stöchiometrischen Menge eines 2-Amino-1,3,5-triazinderivats III 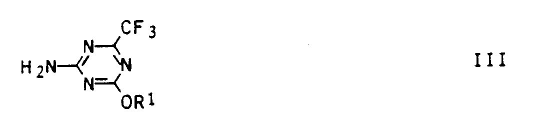 umsetzt.

3. Verfahren zur Herstellung der [[(1,3,5-Triazin-2-yl)aminocarbonyl]aminosulfonyl]benzoesäureester der Formel I gemäß Anspruch 1, dadurch gekennzeichnet, daß man ein entsprechendes Sulfonamid der Formel IV 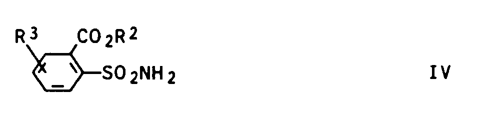 in an sich bekannter Weise in einem inerten organischen Lösungsmittel mit einem Phenylcarbamat V 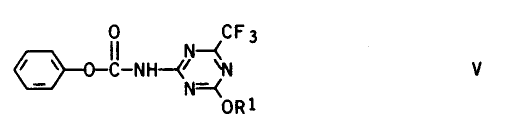 oder mit einem Isocyanat der Formel VI 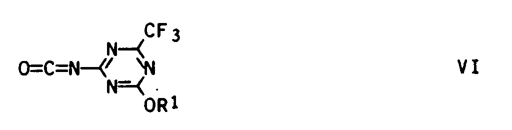 umsetzt.

4. Verfahren zur Herstellung von Salzen der Verbindungen I gemäß Anspruch 1, dadurch gekennzeichnet, daß man eine Verbindung der Formel I gemäß Anspruch 1 in an sich bekannter Weise in Wasser oder einem inerten organischen Lösungsmittel mit einer Base deprotoniert.

5. Herbizides Mittel, enthaltend einen [[(1,3,5-Triazin-2-yl)aminocarbonyl]aminosulfonyl]benzoesäureester der Formel I gemäß Anspruch 1 oder sein Salz sowie hierfür übliche Trägerstoffe.

6. Verfahren zur Bekämpfung unerwünschten Pflanzenwuchses, dadurch gekennzeichnet, daß man eine herbizid wirksame Menge eines [[(1,3,5-Triazin-2-yl)aminocarbonyl]aminosulfonyl]benzoesäureesters der Formel I gemäß Anspruch 1 oder eines seiner Salze auf die Pflanzen und/oder ihren Lebensraum einwirken läßt.

7. Verwendung der Verbindungen I gemäß Anspruch 1 als herbizide Mittel.

## Claims

1. A [[(1,3,5-triazin-2-yl)aminocarbonyl]aminosulfonyl]benzoic ester of the formula I 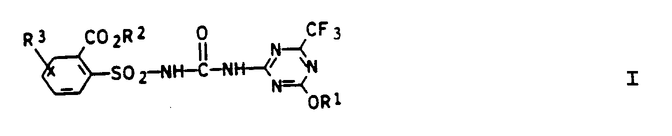 where
R¹ is methyl or ethyl;
R² is i-propyl;
R³ is hydrogen, fluorine, chlorine, methyl or methoxy,
and the salts thereof which can be used in agriculture.

2. A process for preparing a [[(1,3,5-triazin-2-yl)aminocarbonyl]aminosulfonyl]benzoic ester of the formula I as claimed in claim 1, which comprises reacting a sulfonyl isocyanate II 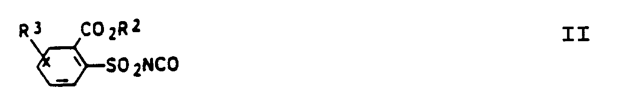 in a conventional manner in an inert organic solvent with approximately the stoichiometric amount of a 2-amino-1,3,5-triazine derivative III

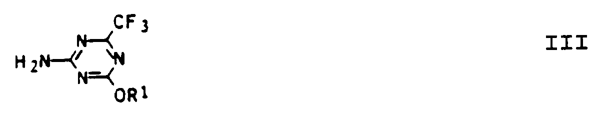

3. A process for preparing a [[(1,3,5-triazin-2-yl)aminocarbonyl]aminosulfonyl]benzoic ester of the formula I as claimed in claim 1, which comprises reacting a sulfonamide of the formula IV 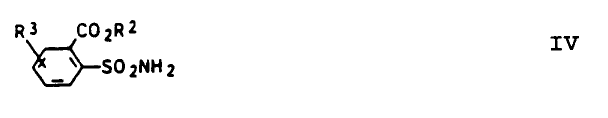 in a conventional manner in an inert organic solvent with a phenyl carbamate V 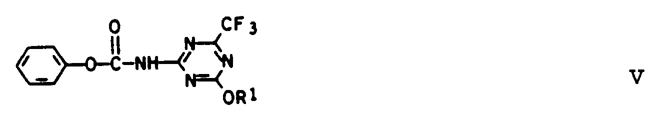 or with an isocyanate of the formula VI

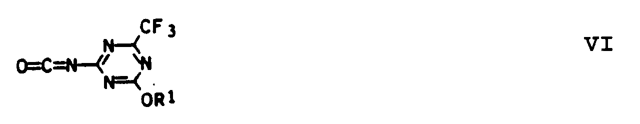

4. A process for preparing a salt of a compound I as claimed in claim 1, which comprises deprotonating a compound of the formula I as claimed in claim 1 in a conventional manner in water or in an inert organic solvent with a base.

5. A herbicidal agent containing a [[(1,3,5-triazin-2-yl)aminocarbonyl]sulfonyl]benzoic ester of the formula I as claimed in claim 1 or its salt and carriers conventional for this purpose.

6. A method for controlling unwanted plant growth, which comprises exposing the plants and/or their habitat to a herbicidally effective amount of a [[(1,3,5-triazin-2-yl)aminocarbonyl]sulfonyl]benzoic ester of the formula I as claimed in claim 1 or one of its salts.

7. The use of the compounds I as claimed in claim 1 as herbicidal agents.

## Revendications

1. Esters d'acide [[(1,3,5-triazin-2-yl)aminocarbonyl]amino-sulfonyl]benzoïque de formule générale I 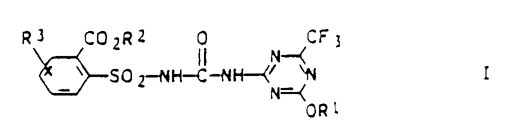 dans laquelle les substituants ont les significations suivantes :
R¹ un groupe méthyle ou éthyle
R² i-propyle
R³ hydrogène, fluor, chlore, méthyle ou méthoxy
ainsi que leurs sels utilisables en agriculture.

2. Procédé de préparation d'esters d'acide [[(1,3,5-triazin-2-yl)aminocarbonyl]amino-sulfonyl]benzoïque de formule générale I selon la revendication 1, caractérisé par le fait que l'on fait réagir un isocyanate de sulfonyle II 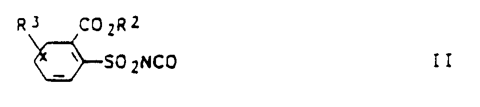 de manière connue en soi, dans un solvant organique inerte avec la quantité à peu près stoechiométrique d'un dérivé de 2-amino-1,3,5-triazine III

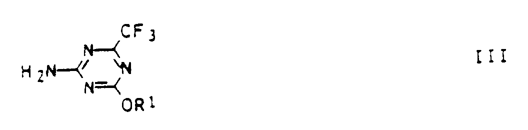

3. Procédé de préparation d'esters d'acide [[(1,3,5-triazin-2-yl)aminocarbonyl]amino-sulfonyl]benzoïque de formule générale I selon la revendication 1, caractérisé par le fait que l'on fait réagir un sulfonamide correspondant de formule IV 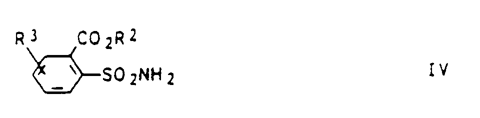 de manière connue en soi, dans un solvant organique inerte avec un carbamate de phényle V 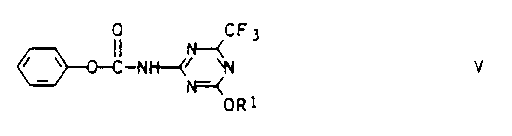 ou avec un isocyanate de formule VI

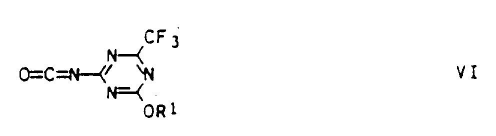

4. Procédé de préparation de sels des composés I selon la revendication 1, caractérisé par le fait que l'on déprotonise un composé de formule I selon la revendication 1 avec une base, de manière connue en soi, dans de l'eau ou un solvant organique inerte.

5. Herbicide contenant un esters d'acide [[(1,3,5-triazin-2-yl)aminocarbonyl]amino-sulfonyl]benzoïque de formule générale I selon la revendication 1 ou son sel ainsi que des supports usuels pour cela.

6. Procédé de lutte contre la croissance de plantes indésirables, caractérisé par le fait que l'on fait agir une quantité efficace comme herbicide d'un ester d'acide [[(1,3,5-triazin-2-yl)aminocarbonyl]amino-sulfonyl]benzoïque de formule générale I selon la revendication 1 ou un de ses sels, sur les plantes et/ou leur biotope.

7. Utilisation des composés I selon la revendication 1 comme herbicide.
